# EUROPEAN PATENT SPECIFICATION

(11) **EP 0 918 796 B1**
(45) Date of publication and mention of the grant of the patent: **10.01.2007**
(21) Application number: 97938077.1
(22) Date of filing: 01.08.1997
(51) Int. Cl.: C07K 16/12, C12N 5/12, G01N 33/569, A61K 39/395

(54) **MONOCLONAL ANTIBODY WHICH AGGLUTINATES E. COLI HAVING THE CS4-CFA/I FAMILY PROTEIN**
MONOKLONALER ANTIKÖRPER FÜR DIE AGGLUTINIERUNG VON E. COLI, DAS EIN PROTEIN DER CS4/CFA-FAMILIE ENTHÄLT
ANTICORPS MONOCLONAL PERMETTANT D'AGGLUTINER E. COLI POSSEDANT UNE PROTEINE DE LA FAMILLE CS4-CFA/I

(30) Priority: 02.08.1996 US 23075 P
(43) Date of publication of application: 02.06.1999
(73) Proprietor: DEPARTMENT OF THE ARMY, U.S. GOVERNMENT, Frederick, MD 21702-5012 (US); Virion Systems, Inc., Rockville, MD 20850 (US)
(72) Inventor: CASSELS, Frederick, Ellicott City, MD 21043 (US); LEES, Andrew, Silver Spring, MD 20910 (US); SCHUMAN, Richard, Gaithersburg, MD 20878 (US)
(74) Representative: Harding, Charles Thomas
(86) International application number: PCT/US1997/013477
(87) International publication number: WO 1998/005687

(56) References cited:
- WO-A-92/01703
- WO-A-96/38171
- CASSELS F J ET AL: "ANTIBODY TO N-TERMINAL CONSENSUS PEPTIDE IS CROSS-REACTIVE WITH ALLSIX MEMBERS OF THE ENTEROTOXIGENIC E. COLI CFA/I FAMILY" , PAP. JT. MEETING US-JP. COOP. MED. SCI. PROGRAM PANELS MALNULTR. CHOLERA, PUBL. 1997, MEETING DATE 1995, PAGE(S) 275-279 XP000775950 * the whole document *
- INFECTION AND IMMUNITY, October 1994, Vol. 62, No. 10, RUDIN et al., "Monoclonal Antibodies Against Enterotoxigenic Escherichia Coli Colonization Factor Antigen I (CFA/I) that Cross-React Immunologically with Heterologous CFAs", pages 4339-4346.
- VACCINE, 1993, Vol. 11, No. 2, GREWAL et al., "Induction of Colonization Factor Antigen I (CFA/I) and Coli Surface Antigen 4 (CS4) of Enterotoxigenic Escherichia Coli: Relevance for Vaccine Production", pages 221-226.
- JOURNAL OF CLINICAL MICROBIOLOGY, October 1988, Vol. 26, No. 10, LOPEZ-VIDAL et al., "Monoclonal Antibodies Against Different Epitopes on Colonization Factor Antigen I of Enterotoxin-Producing Escherichia Coli", pages 1967-1972.
- JOURNAL OF CLINICAL MICROBIOLOGY, March 1993, Vol. 31, No. 3, VIBOUD et al., "Characterization of Monoclonal Antibodies Against Putative Colonization Factors of Enterotoxigenic Escherichia Coli and Their Use in an Epidemiological Study", pages 558-564.
- JOURNAL OF CLINICAL MICROBIOLOGY, August 1993, Vol. 31, No. 8, GHOSH et al., "Evaluation of Conventional Media for Detection of Colonization Factor Antigens of Enterotoxigenic Escherichia Coli", pages 2163-2166.
- EUR. J. BIOCHEM., 1982, Vol. 124, KLEMM, "Primary Structure of the CFA1 Fimbrial Protein from Human Enterotoxigenic Escherichia Coli Strains", pages 339-348.
- INFECTION AND IMMUNITY, June 1992, Vol. 60, No. 6, CASSELS et al., "Analysis of Escherichia Coli Colonization Factor Antigen I Linear B-Cell Epitopes, as Determined by Primate Responses, Following Protein Sequence Verification", pages 2174-2181.
- INFECTION AND IMMUNITY, January 1989, Vol. 57, No. 1, WOLF et al., "Characterization of CS4 and CS6 Antigenic Components of PCF8775, a Putative Colonization Factor Complex from Enterotoxigenic Escherichia Coli E8775", pages 164-173.

### SEQUENCE LISTING

<110> Dept of Army US Government
   Virion Systems Inc
<120> Monoclonal antibody which agglutinates E.coli having the CS4-CFA/1 family protein
<130> p644ep
<140>
   <141>
<160> 3
<170> PatentIn Ver. 2.1
<210> 1
   <211> 36
   <212> PRT
   <213> E.coli
<400> 1
   Val Glu Lys Asn Ile Thr Val Thr Ala Ser Val Asp Pro Thr Ile Asp
    1               5                  10                  15
   Leu Leu Gln Ala Asp Gly Ser Ala Leu Pro Ser Ala Val Ala Leu Thr
                20                  25                  30
   Tyr Ser Pro Ala
            35
<210> 2
   <211> 8
   <212> PRT
   <213> E.Coli
<400> 2
   Ser Ala Val Ala Leu Thr Tyr Ser
   1 5
<210> 3
   <211> 37
   <212> PRT
   <213> E. Coli
<400> 3
   Cys Val Glu Lys Asn Ile Thr Val Thr Ala Ser Val Asp Pro Thr Ile
   1 5 10 15
   Asp Leu Leu Gln Ala Asp Gly Ser Ala Leu Pro Ser Ala Val Ala Leu
   20 25 30
   Thr Tyr Ser Pro Ala
   35

## Description

### Field of the Invention:

This invention relates to a monoclonal antibody to a consensus peptide of the formula:
VEKNITVTASVDPTIDLLQADGSALPSAVALTYSPA . The monoclonal antibody of the invention binds exclusively to the sequence SAVALTYS.

### Background of the invention:

The effect of E. coli in mammals is dependent on the particular strain of organism. Many beneficial E. coli are present in the intestines. Since the initial association of E. coli with diarrheal illness, five categories of diarrheagenic E. coli have been identified and are presently recognized: enterotoxigenic (ETEC), enteropathogenic (EPEC), enterohemorrhagic (EHEC), enteroaggregative (EAggEC), and enteroinvasive (EIEC). These categories are grouped according to characteristic virulence properties, such as elaboration of toxins and colonization factors and/or by specific types of interactions with intestinal epithelial cells. ETEC are the most common of the diarrheagenic E. coli and pose the greatest risk to travelers. E. coli of the family CS4-CFA/I are some of the more common enterotoxigenic E. coli. There is need for vaccines which are specific against this class of E. coli that give rise to antibodies that cross-react with and cross-protect against the more common members of the CS4-CFA/I family. Six members of this family of ETEC fimbrial proteins are CFA/I, CS1, CS2, CS4, CS17 and PCF 0166. ETEC are responsible for high infant mortality in developing countries, with an estimate that almost 800,000 deaths per year due to these organisms. These organisms also cause illness in adult travelers to regions where the disease is endemic.

Colonization factor antigens (CFA) of ETEC are important in the initial step of colonization and adherence of the bacterium to intestinal epithelia. In epidemiological studies of adults and children with diarrhea, CFA/I is found in a large percentage of morbidity attributed to ETEC. The CFA/I is present on the surfaces of bacteria in the form of pili (fimbriae), which are rigid, 7 nm diameter protein fibers composed of repeating pilin subunits. The CFA/I antigens promote mannose-resistant attachment to human brush borders with an apparent sialic acid sensitivity.

A study of proteins in E. coli belonging to the CS4-CFA/I family resulted in the finding that the H-terminal region of the protein maintains a high degree of sequence identity between members of this group. Immunological evidence shows that cross-reaction exists between members of the family CS4-CFA/I.
Lopez-Vidal *et al*. (*Journal of Clinical Microbiology*, October 1998, page 1967-1972) describe monoclonal antibodies against different epitopes on colonisation factor antigen I enterotoxin-producing *Escherichia coli*.
Cassels *et al*. describe that an antibody to N-teiminal consensus peptide is cross-reactive with all six members of the enterotoxigenic E.coli CFA/I family, in *Cytokines*, *cholera and the gut [papers from the joint meeting of the United States-Japan cooperatite medical sciences program panels on malnutrition and cholera]*, Kiawah Island S.C., Nov. 30-Dec-3, 1995. Reported 1997, Editors: G. Keusch & M. Kawakami, publisher IOS Press, Amsterdam, The Netherlands, Pages 275-279.
Rudin *et al.* describe monoclonal antibodies against enterotoxigenic *Escherichia coli* colonisation factor antigen I (CFA/I) that cross react immunulogically with heterologous CFAs *(Infection and Immunity,* vol 62, No. 10, October 1994 pages 4339-4346).

Cassels, et al. have identified a consensus peptide of 36 amino acids which acts as an immunogen raising antibodies against the proteins of all members of the E. coli family CS4,-CFA/I . The region of the protein represented in the subunit encompasses known linear B- and T-cell epitopes of CFA/I. The consensus peptide has a high level of homology to strains bearing six different colonization factors. The consensus peptide is of the formula:
VEKNITVTASVDPTIDLLQADGSALPSAVALTYSPA

It is the purpose of this invention to identify a monoclonal antibody raised to the consensus peptide of Cassels and which will agglutinate all bacteria bearing CS4-CFA/I family proteins.

### Description of the Invention:

A monoclonal antibody produced by the hybridoma with deposit number ATCC HB12163 which agglutinates all *Escherichia coli* bacteria bearing the CS4-CFA/I family proteins CFA/1, CS1, CS2, CS4, CS17 or PCF0166.

### Preparation of the Immunogen:

### A: Iodoacetylation of tetanus toxoid:

To 0.64 ml of a composition containing 18.9 mg/xul (12 mg) of tetanus toxoid (TT) (obtained from SmithKline Beecham) was added 5x HEPES buffer (75 µl of 0.75 M HEPES, 5 mM EDTA, pH 7.3). The TT was labeled with a 40 fold molar excess of N-hydroxysuccinimidyl iodoacetate (32 µl of 0.1 mM in dimethylformamide). After two hours, the protein was desalted on 2 P6 cartridges (BioRad) in series, equilibrated with HEPES buffer (0.15M HEPES, 1mM EDTA, pH 7.3). The void volume fraction was concentrated to about 0.7 ml using a Macrosep 50 device (Filtron Corp).

### B: Reduction of peptide:

About 10 mg of peptide consensus peptide of the formula
CVEKNITVTASVDPTIDLLQADGSALPSAVALTYSPA was solubilized in 1.1 ml HEPES buffer containing 100 µl acetonitrile and reduced by the addition of solid dithiothreitol to a final concentration of 0.5 M. After 1 hour the peptide was desalted in two parts on a 1 x 50 cm G10 column (Pharmacia), equilibrated with acetate buffer (10 mM sodium acetate, 0.1 M NaCl, 2 mM EDTA and 0.02% sodium azide at pH 5) and run at 1 ml/min. The void volume fractions were pooled. Ellman's reagent (G.L. Ellman, Arch Biochem & Biophys., 82:70 (1959)) was used to determine that the peptide was reduced to a thiol.

### C: Coupling of peptide to Tetanus toxoid:

Six ml of the reduced peptide was added to 0.3 ml of TT labeled with N-hydroxysuccinimidyl iodoacetate and 1 ml 5x HEPES buffer. After overnight incubation at 4°C, the conjugate was concentrated to about 1 ml using a Macrosep 50 device, then desalted into HEPES buffer using P6 cartridges, concentrated again (Macrosep 50), and, finally, filtered through a 0.45 micron Millex HV filter (Millipore). Evaluation of the protein content using the BioRad assay showed total protein content to be about 2.6 mg/ml.

### Monoclonal Antibody Production:

### A: Preparation of anticonsensus peptide monoclonal antibody:

Six BALB/c mice identified as numbers 8378-8383, were immunized with the consensus peptide-TT conjugate. On designated day 1, each mouse was injected subcutaneously with 25 µg conjugate in 0.2 ml emulsified in 60% complete Freund's adjuvant. On day 23, a serum sample was obtained from each mouse. On day 35, all mice except #8382 received a boost of 10 *µ*g consensus peptide conjugate in 0.2 ml 60% incomplete Freund's adjuvant. Mouse 8382 was given 10 *µ*g conjugate of the peptide in 0.1 ml phosphate-buffered saline (PBS).

On day 37, mouse 8382 was used for fusion (96-104). This fusion did not result in production of a monoclonal anti-consensus peptide.

On day 82, the mice received booster immunizations of 10 µg consensus peptide conjugate in 0.2 ml emulsified in 60% incomplete Freund's adjuvant.

On day 85, the spleen from mouse # 8383 was fused with FOX-NY myeloma wherein the myeloma population viability was 97.4%. 1.36 x 10⁸ spleen cells were fused with 1.37 x 10⁷ myeloma cells, using PEG (1400 molecular weight) as a fusogen. The hybridomas were assigned culture number 96-109.

Hybridomas were planted into 8 96-well tissue culture dishes with 100 µl/well in RPMI 1640 supplemented with 10% heat-inactivated fetal bovine serum, 10% Hybridoma Serum Free Media (SFM), 100 µM hypoxanthine and 16 µM thymidine (the hypoxanthine and thymidine combination being referred to herein as HT). Eight wells were also planted with FOX-NY myeloma cells only (no hybridomas) as a control. All samples were incubated at 37°C in a humidified atmosphere of 5% CO₂ in air. After 24 hours, all wells received 100 *µ*l RPMI 1640 supplemented with 10% heat-inactivated fetal bovine serum, 10% hybridoma SFM, 200 *µ*M hypoxanthine, 0.8 *µ*M aminopterin and 32 *µ*M thymidine. (The hypoxanthine, aminopterin and thymidine combination being referred to herein as HAT.)

Approximately 96 hours after the fusion, the FOX-NY myelomas in control wells appeared to be dead. Many other wells contained growing colonies of hybridomas seven days after fusion. The growing cells were fed by addition of RPMI 1640 supplemented with 10% heat-inactivated fetal bovine serum, 10% hybridoma SFM and HT. Four days thereafter, the supernatants were tested for the presence of anti-consensus peptide antibodies.

For analysis of peptide binding, an ELISA was used. Nunc Maxisorp stripwells were coated overnight at room temperature with 100 µl/well of consensus peptide at 1 µg/ml PBS. The wells were then washed four times with PBS containing 0.05% Tween-20 (PBS-T) to remove unbound material. Each well then received 50 µl of PBS-T. Fifty µl of supernatant was then transferred from the cell culture plate to the corresponding wells of the immunoassay dish. Prior to the transfer of the cell culture, wells were screened microscopically to identify wells without hybridomas. One such well from each plate was used as a background control by substituting PBS-T or medium for the culture supernatant. The plates were then sealed and incubated for 30 to 60 minutes at room temperature in the dark in a draft-free environment. The wells were thereafter washed four times with PBS-T to remove unbound material. Each well then received 95 *µ*l of sheep anti-mouse IgG-HRP (horse radish peroxidase), diluted 1:10000 in PBS-T. Following a 30 minute incubation, the wells were again washed and each well received 100 µl of tetramethylbenzidine (TMB) substrate solution. The plates were then incubated in the dark for 15 minutes at room temperature, after which the reactions were stopped by addition of 80 *µ*l of TMB Stop Solution. The absorbance of each well was determined at 450 mn using a Molecular Devices microplate reader.

Absorbance values for 32 of the supernatants from wells with growing hybridomas was greater than 0.200 units. Of these, only two wells, designated CA8 (1.743) and FE8 (1.092) had absorbance values of greater than 1.000. All thirty-two cultures were expanded by transfer into 24-well culture dishes and grown on RPMI 1640 with 10% FBS. Upon retest, only colony FE8 continued to produce antibodies reactive with the consensus peptide. This culture was expanded to growth in a T75 culture flask and samples were cryopreserved.

The isotype of the antibody secreted by 96-109FE8 was determined using a Zymed isotype kit. The results indicated that the antibody was an IgM with a kappa light chain. The 96-109FE8 culture was cloned into 96-well culture dishes by diluting the cells to a concentration of 4.5-5 cells/ml in RPMI 1640 with 20% FBS and 10% hybridoma SFM. Each well received 200 µl of the cell suspension. Each well was checked for the presence of a single focus of growing hybridomas. The supernatants from each such well were tested for binding of the antibody to the consensus peptide epitope. All of the supernatants were active, suggesting that all of the surviving cells in the original culture were secretors of the antibody of interest, and that the genotype was stable. One clone, designated 96-109FE8 IH11, was expanded, cryopreserved and used in the production of ascites.

### Testing of hybridoma tissue culture supernatant for agglutinating activity:

Bacterial culture: ETEC strains bearing the colonization factors CFA/I, CS1, CS2 and CS4 were grown overnight at 37°C on colonization factor antigen agar [10 gm Casamino acids, 1% (Difco Laboratories, Detroit, MI); 1.5 gm yeast extract (Difco), 0.15%; 0.1 gm MgSO₄·7H₂O), 0.005% (Sigma, St. Louis); 0.008 gm MnCl₂, 0,0005% MnCl₂ (Sigma); 20 gm agar (Difco);, q.s. to 1 liter with deionized water]. Those ETEC strains bearing the colonization factors CS17 and PCF 0166 are also grown on colonization factor antigen agar, which was also supplemented with 0.15% bile salts (bile salts #3, Difco). Bacteria were harvested into phosphate buffer saline (PBS) solution and the concentration of bacterial suspension was adjusted to an optical density of 20 (when diluted 1/20 gives an OD of 1.00+/- 0.005 at 600 nm). Bacterial culture supernatant was tested at full strength or serially diluted 1:2 with PBS.

The following assay was used: Eight *µ*l of bacterial suspension was mixed with an equal volume of tissue culture supernatant dilution on a glass microscope slide (25 x 75 mm) at room temperature. In a separate place on the same slide there is a control consisting of bacterial suspension with 8 µl of PBS (autoagglutination control). The mixture is rocked back and forth continuously and the agglutination is observed at 10 seconds, 30 seconds, 1 minute and 2 minutes. The results are visually scored as follows:
4 = agglutination in less than 10 seconds with large clumps
3 = agglutination in less than 30 seconds with large clumps
2 = agglutination in less than 60 seconds with medium clumps
1 = agglutination in less than 2 minutes with small clumps
0 = no agglutination within 2 minutes.

### Results:

At undiluted tissue culture supernatant (estimated at 1 µg/ml of antibody), no bacterial strains were agglutinated. After concentration of tissue culture supernatant to 20 fold concentration (YM 100 centrifugal ultrafilter, Amicon, Danvers, Massachusetts), only the bacterial strain expressing CFA/I was agglutinated (H10407NM). The monoclonal antibody supernatant was then concentrated 130 fold from original strength and tested. Under these circumstances, the antibody agglutinated all bacteria bearing CS4-CFA/I family proteins.

The hybridoma identified as 96-109FE8 IH11 has been deposited in the American Type Culture Collection in Rockville, Maryland and given the designation ATCC HB-12163.

As indicated above the antibody may be used for purposes of identifying E. coli bearing the CS4-CFA/I protein family. The samples suspected of containing E. coli of the CS4-CFA/I protein may be grown by usual methods in the clinical laboratory. The colonies of organisms may then be suspended by the method disclosed above. The suspended organisms are then exposed to a composition containing at least 30 *µ*g/ml of antibody. In a preferred embodiment, the suspended organisms would be exposed to a composition containing an antibody concentration of 100 to 130 *µ*g/ml. Appropriate samples would include stools from patients suffering from diarrhea and for testing food and environmental samples for contamination with ETEC E. coli organisms.

The monoclonal antibody (MAB) is useful for identifying members of the CS4-CFA/I family in cultures. Assay kits containing the MAB may be prepared and may contain, in addition to the MAB of the invention, agents for tagging for facilitate identification of the MAB/antigen complex. Such tags include radioactive isotopes, fluorescing agents and colorometric indicators. Such agents may be attached to solid supports. For example, an ELISA test kit system may be used to identify the MAB/antigen complex.

Compositions containing the MAB of the invention may be prepared using as a carrier appropriate for addition to a growth media. Saline and other buffered solutions known in the art are appropriate as carriers for the MAB.

MABs of the invention may also be prepared in pharmaceutically acceptable carrier solutions and may be administered to the infected area to agglutinate the bacteria bearing CS4-CFA/I proteins. Administration would provide means for the compositions to contact the organisms. For example, the compositions could be administred orally in capsules which protect the antibody from distruction in the stomach and duedenum. The compositions are appropriate for use both for short-term prophylaxis and for treatment of ETEC E. coli infections by administration of an ETEC E. coli agglutinating effective amount of the pharmaceutical composition.

## Claims

1. A monoclonal antibody produced by the hybridoma with deposit number ATCC HB12163 which agglutinates all *Escherichia coli* bacteria bearing the CS4-CFA/I family proteins CFA/I, CS 1, CS2, CS4, CS 17 or PCF0166.

2. A composition of matter comprising a monoclonal antibody of claim 1 in a carrier.

3. A composition according to claim 2, wherein the carrier is appropriate for application to a bacteria-containing growth media.

4. A composition according to claim 2, wherein the carrier is a pharmaceutically acceptable carrier.

5. A composition according to claim 2, wherein the carrier is saline.

6. The monoclonal antibody of claim 1, wherein the monoclonal antibody has a tag to assist in identification of a complex between the monoclonal antibody and its antigen.

7. The composition according to claim 2, wherein the monoclonal antibody has a tag to assist in identification of a complex between the monoclonal antibody and its antigen.

8. The monoclonal antibody of claim 6 or the composition of claim 7, wherein the tag is a radioactive isotope, a fluorescing agent or a calorimetric tag.

9. An assay for detecting the presence of a member of CS4-CFA/I member proteins in a sample which comprises contacting the sample with the monoclonal antibody according to claim 1, and detecting the presence or absence of a complex between the monoclonal antibody and any proteins in the sample.

10. Use of the monoclonal antibody according to the composition of claim 4 in the manufacture of a medicament for treating an illness arising from infection with bacteria bearing the CS4-CFA/I family proteins.

11. Use of the monoclonal antibody according to the composition of claim 4 in the manufacture of a medicament for prophylaxing against an illness arising from infection with bacteria bearing the CS4-CFA/I family proteins.

12. A kit comprising the monoclonal antibody of claim 1 and a tag for identifying a complex between the monoclonal antibody and its antigen.

13. The monoclonal antibody of claim 12, wherein the tag is a radioactive isotope, a fluorescing agent or a calorimetric tag.

## Patentansprüche

1. Monoklonaler Antikörper, hergestellt durch das Hybridom mit der Hinterlegungsnummer ATCC HB12163, welcher alle *Escherichia coli*-Bakterien, die die Proteine CFA/I, CS1, CS2, CS4, CS17 oder PCF0166 der CS4-CFA/I-Familie tragen, agglutiniert.

2. Stoffzusammensetzung, welche einen monoklonalen Antikörper nach Anspruch 1 in einem Träger umfaßt.

3. Zusammensetzung nach Anspruch 2, wobei der Träger für die Aufbringung auf ein Bakterien enthaltendes Wachstumsmedium geeignet ist.

4. Zusammensetzung nach Anspruch 2, wobei der Träger ein pharmazeutisch verträglicher Träger ist.

5. Zusammensetzung nach Anspruch 2, wobei der Träger Salzlösung ist.

6. Monoklonaler Antikörper nach Anspruch 1, wobei der monoklonale Antikörper eine Markierung aufweist, um die Identifizierung eines Komplexes zwischen dem monoklonalen Antikörper und seinem Antigen zu unterstützen.

7. Zusammensetzung nach Anspruch 2, wobei der monoklonale Antikörper eine Markierung aufweist, um die Identifizierung eines Komplexes zwischen dem monoklonalen Antikörper und seinem Antigen zu unterstützen.

8. Monoklonaler Antikörper nach Anspruch 6 oder Zusammensetzung nach Anspruch 7, wobei die Markierung ein radioaktives Isotop, ein fluoreszierendes Mittel oder eine kalorimetrische Markierung ist.

9. Assay zum Detektieren des Vorliegens eines Mitglieds der CS4-CFA/I-Proteinfamilie in einer Probe, welcher das Inkontaktbringen der Probe mit dem monoklonalen Antikörper nach Anspruch 1 und das Detektieren des Vorliegens oder Nichtvorliegens eines Komplexes zwischen dem monoklonalen Antikörper und irgendwelchen Proteinen in der Probe umfaßt.

10. Verwendung des monoklonalen Antikörpers gemäß der Zusammensetzung nach Anspruch 4 bei der Herstellung eines Medikaments zur Behandlung einer Erkrankung, die durch eine Infektion mit Bakterien, die die Proteine der CS4-CFA/I-Familie tragen, hervorgerufen wird.

11. Verwendung des monoklonalen Antikörpers gemäß der Zusammensetzung nach Anspruch 4 bei der Herstellung eines Medikaments zur Prophylaxe gegen eine Erkrankung, die durch eine Infektion mit Bakterien, die die Proteine der CS4-CFA/I-Familie tragen, hervorgerufen wird.

12. Kit, welcher den monoklonalen Antikörper nach Anspruch 1 und eine Markierung zum Identifizieren eines Komplexes zwischen dem monoklonalen Antikörper und seinem Antigen umfaßt.

13. Monoklonaler Antikörper nach Anspruch 12, wobei die Markierung eine Markierung mit einem radioaktiven Isotop, eine Markierung mit einem fluoreszierenden Mittel oder eine kalorimetrische Markierung ist.

## Revendications

1. Anticorps monoclonal produit par l'hybridome ayant le numéro de dépôt ATCC HB12163, qui agglutine toutes les bactéries *Escherichia coli* portant les protéines CFA/I, CS1, CS2, CS4, CS17 ou PCF0166 de la famille CS4-CFA/I.

2. Composition de matière comprenant un anticorps monoclonal selon la revendication 1, dans un véhicule.

3. Composition selon la revendication 2, dans laquelle le véhicule est approprié à une application sur un milieu de culture contenant des bactéries.

4. Composition selon la revendication 2, dans laquelle le véhicule est un véhicule acceptable sur le plan pharmaceutique.

5. Composition selon la revendication 2, dans laquelle le véhicule est du sérum physiologique.

6. Anticorps monoclonal selon la revendication 1, dans lequel l'anticorps monoclonal comprend un marqueur pour faciliter l'identification d'un complexe entre l'anticorps monoclonal et son antigène.

7. Composition selon la revendication 2, dans laquelle l'anticorps monoclonal comprend un marqueur pour faciliter l'identification d'un complexe entre l'anticorps monoclonal et son antigène.

8. Anticorps monoclonal selon la revendication 6 ou composition selon la revendication 7, dans lequel le marqueur est un isotope radioactif, un agent fluorescent ou un marqueur calorimétrique.

9. Dosage pour détecter la présence d'un membre des protéines de la famille CS4-CFA/I dans un échantillon, qui comprend la mise en contact de l'échantillon avec l'anticorps monoclonal selon la revendication 1, et la détection de la présence ou de l'absence d'un complexe entre l'anticorps monoclonal et toute protéine dans l'échantillon.

10. Utilisation de l'anticorps monoclonal selon la composition de la revendication 4 dans la fabrication d'un médicament pour traiter une maladie provoquée par une infection avec des bactéries portant les protéines de la famille CS4-CFA/I.

11. Utilisation de l'anticorps monoclonal selon la composition de la revendication 4 dans la fabrication d'un médicament pour la prophylaxie d'une maladie provoquée par une infection avec des bactéries portant les protéines de la famille CS4-CFA/I.

12. Trousse comprenant l'anticorps monoclonal selon la revendication 1 et un marqueur pour identifier un complexe entre l'anticorps monoclonal et son antigène.

13. Anticorps monoclonal selon la revendication 12, dans lequel le marqueur est un isotope radioactif, un agent fluorescent ou un marqueur calorimétrique.
